# EUROPEAN PATENT SPECIFICATION

(11) **EP 3 831 347 B1**
(45) Date of publication and mention of the grant of the patent: **24.01.2024**
(21) Application number: 20210132.5
(22) Date of filing: 26.11.2020
(51) Int. Cl.: A61F 9/00, A61F 9/007

(54) **IMPROVED MEDICAL DEVICE FOR GLAUCOMA SURGERY WITH CONTROLLED AND MODULABLE FILTRATION**
VERBESSERTE MEDIZINISCHE VORRICHTUNG FÜR DIE GLAUKOMCHIRURGIE MIT KONTROLLIERTER UND MODULIERBARER FILTRATION
DISPOSITIF MEDICAL AMELIORE POUR LA CHIRURGIE DU GLAUCOME AVEC FILTRATION CONTROLEE ET MODULABLE

(30) Priority: 05.12.2019 IT 201900023106
(43) Date of publication of application: 09.06.2021
(73) Proprietor: Cecchini, Paolo, 34132 Trieste (IT); Zovatto, Luigino, 07020 Loiri Porto San Paolo, Sassari (IT)
(72) Inventor: Cecchini, Paolo, 34132 Trieste (IT); Zovatto, Luigino, 07020 Loiri Porto San Paolo, Sassari (IT)
(74) Representative: Mitola, Marco

(56) References cited:
- WO-A1-2016/168686
- US-A1- 2004 073 156
- US-B1- 6 186 974
- US-B2- 7 481 816

## Description

### FIELD OF APPLICATION

The present invention relates to an improved medical device for open-angle glaucoma surgery with controlled and modular filtration.

### PRIOR ART

As is known, glaucoma is an ocular disease characterized by the progressive loss of retinal ganglion cells and their axons. The functional consequence is the progressive appearance and subsequent deepening of scotomatous areas (i.e. areas of reduced light sensitivity) to the visual field up to the complete loss of the visual function itself. There are numerous types of glaucoma. The most common is chronic open-angle glaucoma. This form of glaucoma is characterized by high ocular pressure, a wide irido-sclero-corneal junction and damage to the nerve fibers that make up the optic nerve.

The cause is not yet fully known. However, there is evidence of the involvement of some genes. The pathogenetic mechanism involves a spongiform structure called trabeculate, located at the level of the irido-sclero-corneal junction, responsible for allowing the aqueous humor to flow towards Schlemm's canal and from this into the venous stream. In primary open-angle glaucoma there would be an impediment at the level of the trabeculae, which, by preventing the correct drainage of the aqueous humor, would induce an increase in endo-ocular pressure. The hypertonicity would cause the death by apoptosis of the ganglion cells that make up the optic nerve. There is therefore a thinning of the so-called neural rim of the optic nerve, which appears pale and excavated, with progressive and irreversible damage to the visual field. Similar, but often more aggressive, are the forms of pseudoexfoliative and pigmentary glaucoma. Pseudoexfoliative glaucoma is characterized by the presence of whitish furfural material, while pigmentary glaucoma is characterized by the presence of pigment granules. Both phenomena can induce an increase in intraocular pressure since the exfoliated or dispersed material is deposited in the irido-sclero-corneal junction at the level of the trabeculae, preventing the correct flow of the aqueous humor.

The first approach to the treatment of glaucoma consists in the instillation of hypotonizing eye drops. Some molecules reduce the production of aqueous humor, while others promote the outflow thereof. Another therapeutic possibility consists in trabeculoplasty laser, that is a laser treatment at the trabecular level, which aims to increase the outflow of aqueous humor.

When medical therapy based on hypotonizing eye drops is not able to control the progression of glaucomatous damage, a surgical approach can be performed. The surgical techniques are many and differ between filtering and non-filtering. Among the filtering techniques, the Gold Standard of glaucoma surgery is trabeculectomy, proposed by Cairns in the 60s of the last century. In this technique, an opening of the bulbar conjunctiva (usually the upper one), with the base facing the conjunctival fornix or the sclero-corneal limbus, the sculpting of a scleral flap usually of square shape, of the size of about 4x4mm (but the flap may have other shapes, such as triangular or rectangular), the removal of a fragment of trabeculae, the suture of the flap with usually detached sutures, and the suture of the conjunctiva above, are carried out in succession. The removal of the trabeculae allows the aqueous humor to filter from the anterior chamber through the scleral flap to the subconjunctival space, forming the so-called filtering patch. The aqueous humor can then be drained by the venous stream.

An alternative to trabeculectomy is the implantation of a metal device called Ex-Press^{®}. The Ex-Press tube is positioned below the scleral flap in the thickness of the irido-sclero-corneal junction up to the anterior chamber, allowing the filtration of aqueous humor in a similar way to standard trabeculectomy. The device then simulates the effect of trabeculectomy, creating an escape route from the anterior chamber to the subconjunctival space. The major difference between classic trabeculectomy and the implantation of the Ex-Press device is given by the fact that the former is a manual maneuver of the surgeon who physically removes a piece of trabeculae, while the latter consists in the implantation of a metal tube in the trabeculae. Since the diameter of the internal lumen of the device is known (50 or 200 microns depending on the models), filtering surgery with Ex-Press is more standardized, while trabeculectomy is a manual maneuver that the surgeon performs according to his/her own skill, will, and dexterity. It, even if minimally, will always be different from one patient to another. Both surgical techniques have yet another manual phase that is entirely the responsibility of the surgeon, namely the sculpting and subsequent closure, with sutures, of the scleral flap. Each surgeon will make a flap of different shape and size, with variability from surgeon to surgeon (interpersonal) but also from procedure to procedure (intrapersonal). The most critical phase is the suture of the flap, as it determines the amount of filtration allowed by the manual removal of the trabecular block (classic trabeculectomy) or through the Ex-Press device. A further alternative to trabeculectomy is the implantation of another device called InnFocus^{®} MicroShunt: it is a tube with an internal diameter of 70 microns, inserted in the anterior chamber without the need to create the scleral flap, but through a scleral tunnel. It allows the outflow of aqueous humor into the subtenonal space.

Trabeculectomy presents numerous difficulties in post-operative management, among which the possibility of hyperfiltration, with marked hypotonia of the eyeball and the possibility of ex-vacuo choroid haemorrhagic detachment, is underlined. Another postoperative complication is represented by the non-tightness of the conjunctival suture at the level of the filtration patch with the appearance of Seidel's phenomenon. This leakage of aqueous through the suture of the conjunctiva also favors the hypotonia of the bulb and the possibility of postoperative infections given the direct communication between the inside and outside of the eye.

Although a lower rate of complications with Ex-press implantation has been reported in the literature, they may nevertheless occur.

The limits of the techniques described above are:
1) the impossibility of regulating the filtration for the single patient;
2) the impossibility of standardizing surgical maneuvers;
3) unwanted filtration through the suture of the conjunctiva - with possible positive Seidel sign.

The limits 1) and 2) are attributable to the fact that the filtration level is mainly determined by the greater or lesser closure of the scleral flap with the sutures entrusted to the hand of the surgeon. A very tight closure of the scleral flap can cause too little or even no filtration, with hypertonicity and substantial failure of the procedure. On the contrary, a too loose suture of the scleral flap can cause hyperfiltration, with marked post-operative hypotonus and possible hemorrhagic choroid detachment. If left untreated, this complication can lead to irreversible damage to the visual function. Filtration, even in the case of InnFocus^{®} type tube systems, cannot be modulated since the flow of aqueous humor from the anterior chamber to the outside is determined solely by the internal diameter of the device lumen.

The scleral flap can also undergo closure due to scarring. This complication is more often present in cases of too tight suture of the flap itself, with difficulty in filtration, up to its complete closure with failure of the procedure.

As for the limit 3), that is the filtration through the suture of the conjunctiva - positive Seidel sign, the immediate filtration that occurs as soon as the trabeculectomy or the implantation of the Ex-Press or other filtration devices such as the InnFocus^{®} are carried out causes a sudden flow of aqueous humor towards the subconjunctival space. If the conjunctival suture is not perfectly sealed, the possibility of filtering through the suture itself may occur. An undesired and dangerous escape route for aqueous humor is therefore established as it maintains a direct connection between the external environment and the eye, with the possibility of infections up to endophthalmitis. The presence of subconjunctival fluid (aqueous humor) in the immediate postoperative period also delays the healing of the conjunctival surgical wound, especially if it is not tight, as it prevents the correct apposition of the tissues and the healing process thereof. This complication determines the need for very strict clinical controls to monitor the phenomenon and often a return to the operating room for the application of further sutures or the complete remaking of the leaky conjunctival suture. Solutions according to cited prior art are disclosed by US 6186974 B1, US 7481816 B2, US 2004/073156 A1, WO 2016/168686 A1.

### PRESENTATION OF THE INVENTION

Therefore, the need is felt to solve the drawbacks and limitations mentioned above with reference to the prior art.

In particular, the need is felt to overcome the following drawbacks/limitations of the current filtering surgery techniques for open-angle glaucoma:
1) the problem of non-modulable/adjustable/controlled/customizable filtration for the individual patient;
2) the problem of filtration through the conjunctiva towards the outside of the eye in the immediate postoperative period with the onset of the so-called positive Seidel sign, due to the filtration of the aqueous humor through the conjunctival wound not yet sealed;
3) as an effect of the solution of the problem referred to in point 2 (abnormal and undesired filtration through the conjunctival suture) is the resolution in many cases of the postoperative complication of the ocular hypotonus and the consequent onset of possible haemorrhagic detachment of ex vacuo choroid.

Such a need is met by a device according to claim 1.

### DESCRIPTION OF THE DRAWINGS

Further features and advantages of the present invention will be more comprehensible from the following description of preferred embodiments given by way of non-limiting examples, in which:

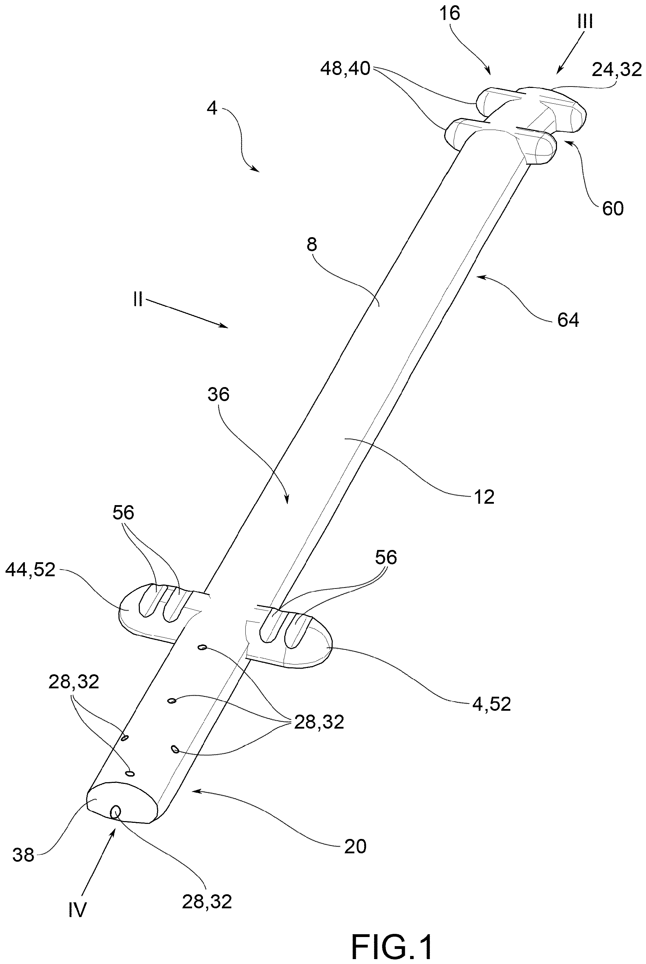

figure 1 shows a perspective view of a medical device for glaucoma surgery according to an embodiment of the present invention;

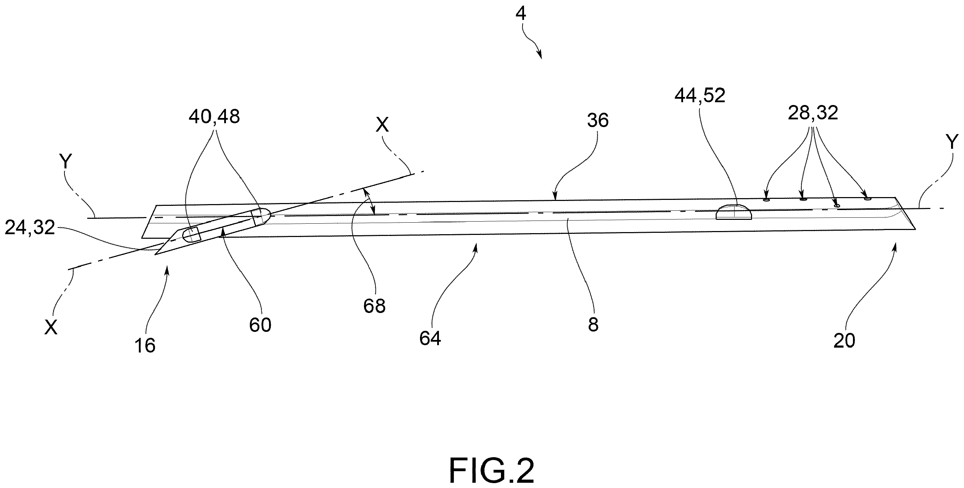

figure 2 shows a lateral view of the device of figure 1 from the side of arrow II indicated in figure 1;

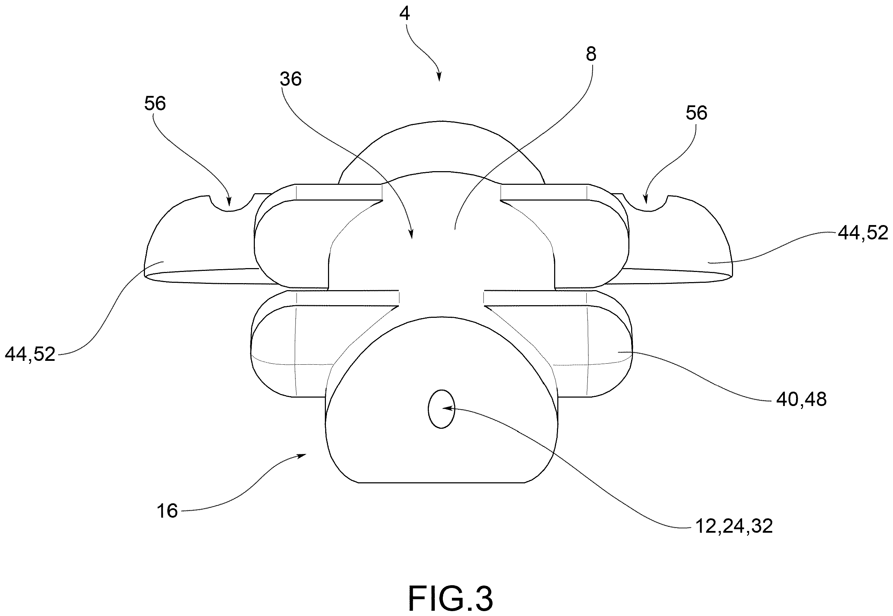

figure 3 is a lateral view of the medical device of figure 1 from the side of arrow III indicated in figure 1;

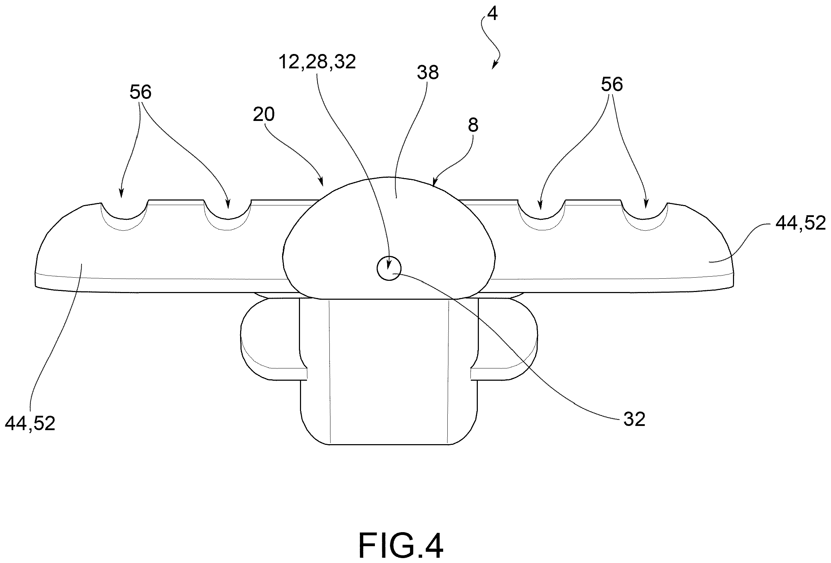

figure 4 is a lateral view of the medical device of figure 1 from the side of arrow IV indicated in figure 1;

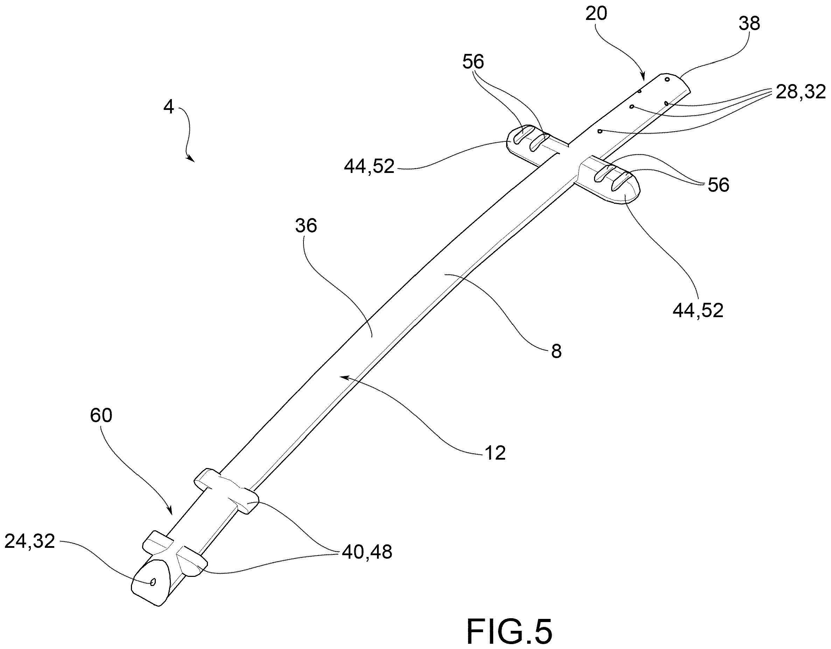

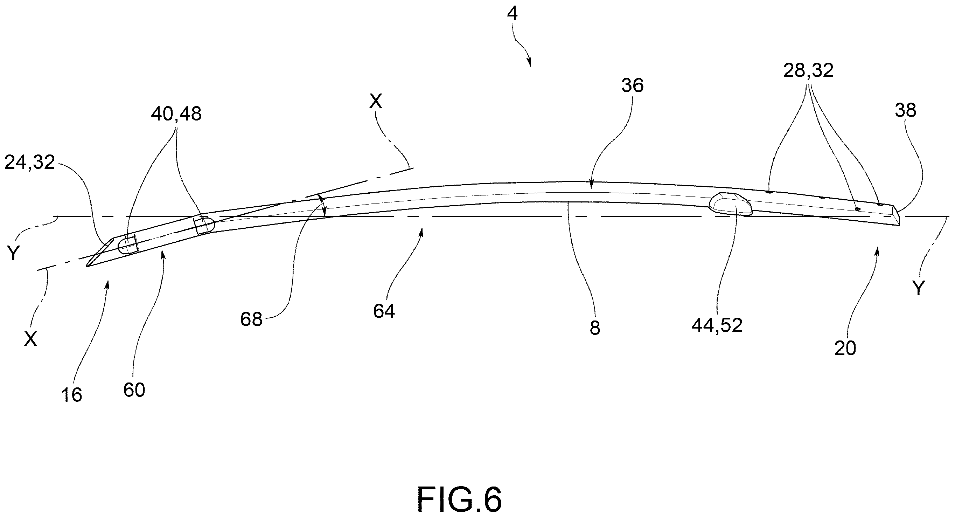

figures 5-6 show a perspective view and a lateral view, respectively, of a medical device for glaucoma surgery according to a further embodiment of the present invention;
figure 7 shows a front perspective view of a medical device for glaucoma surgery according to a further embodiment of the present invention;
figure 8 shows a perspective view of the front detail VIII of the medical device for glaucoma surgery of figure 7;
figure 9 shows a rear perspective view of the medical device for glaucoma surgery of figure 7;
figure 10 shows a top view of the medical device for glaucoma surgery of figure 7;
figure 11 shows a lateral view of the medical device for glaucoma surgery of figure 7;
figures 12a-12b show sectional views of a medical device for glaucoma surgery of the prior art and of a medical device for glaucoma surgery according to the present invention applied to a sclera, respectively.

Elements or parts in common to the embodiments described will be indicated hereafter using the same reference numerals.

### DETAILED DESCRIPTION

With reference to the aforementioned figures, the reference numeral 4 globally indicates a medical device for glaucoma surgery.

The medical device 4 comprises a tubular body 8 provided with a through cavity 12 extending from a proximal or intraocular end 16 to a distal or extraocular end 20. The tubular body 8 may be made of plastic, polymeric, metal, hypoallergenic, biocompatible material and the like.

The tubular body 8 comprises at least one proximal filtration hole 24, located on the side of the proximal end 16, and at least one distal filtration hole 28, located on the side of the distal end 20.

Said proximal and distal filtration holes 24, 28 are fluidically connected to each other by means of the through cavity 12: in other words, the through cavity 12 puts the at least one proximal filtration hole 24, located on the side of the proximal end 16, and the at least one distal filtration hole 28, located on the side of the distal end 20, in fluid communication with each other.

Preferably, the at least one proximal filtration hole 24 is located at the proximal end 16; the at least one distal filtration hole 28 is located on an upper and upper-lateral surface 36 of the tubular body 8. It is also possible to provide that the at least one distal filtration hole 28 is located on a rear surface 38 of the tubular body 8.

By at least one hole it is meant that it is possible to provide a single filtration hole 24, 28 or also a plurality of filtration holes 24, 28. The plurality of filtration holes may comprise a set of holes arranged according to geometric patterns adapted to promote an adequate dispersion of the drained liquid through the through cavity 12.

According to a possible embodiment, the through cavity 12 has a passage width or lumen having an internal diameter between 40 microns and 140 microns.

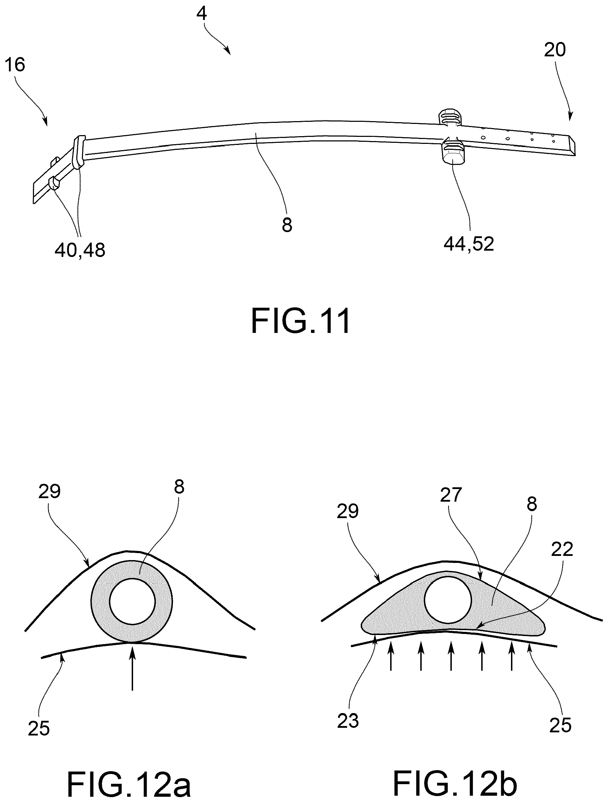

Preferably, the tubular body 8, with respect to a section plane perpendicular to a prevailing direction of extension of the medical device 4 which connects the proximal or intraocular end 16 with the distal or extraocular end 20, has an at least partially concave section 22 at a side or lower face 23 suitable for interfacing with an ocular sclera 25. Figures 12a and 12b illustrate the interaction between the tubular body 8 and the sclera 25 in a solution of the prior art (figure 12a) and in a solution according to the present invention (figure 12b). In the solution of the prior art (figure 12a), the tubular body 8 has a circular section and therefore imprints the underlying sclera 25 in an ideally point-like manner. In this way, the contact pressure is relatively high. On the other hand, in the solution according to the present invention (figure 12b), the tubular body 8 has an at least partially concave section 22 at a side or lower face 23: in this way, the tubular body 8 is able to rest on the sclera 25 in a more uniform manner due to an overall wider support surface, so as to distribute the contact pressure over a wider area of sclera 25.

According to an embodiment of the present invention, the tubular body 8, with respect to a section plane perpendicular to a prevailing direction of extension of the medical device 4 which connects the proximal or intraocular end 16 with the distal or extraocular end 20, has a convex, elliptical or parabolic section, at a side or upper face 27 suitable for interfacing with the conjunctiva 29. Also in this case, with respect to the point-like contact area of the circular solutions of the prior art (figure 12a), the present invention (figure 12b) allows obtaining a more rounded or less steep course of the conjunctiva 29 above and a more uniform contact pressure between the conjunctiva 29 and the upper face 27 of the tubular body 8. A more rounded soft course means a wider curvature that allows increasing the surfaces in mutual contact and therefore decreasing the specific contact pressure between them.

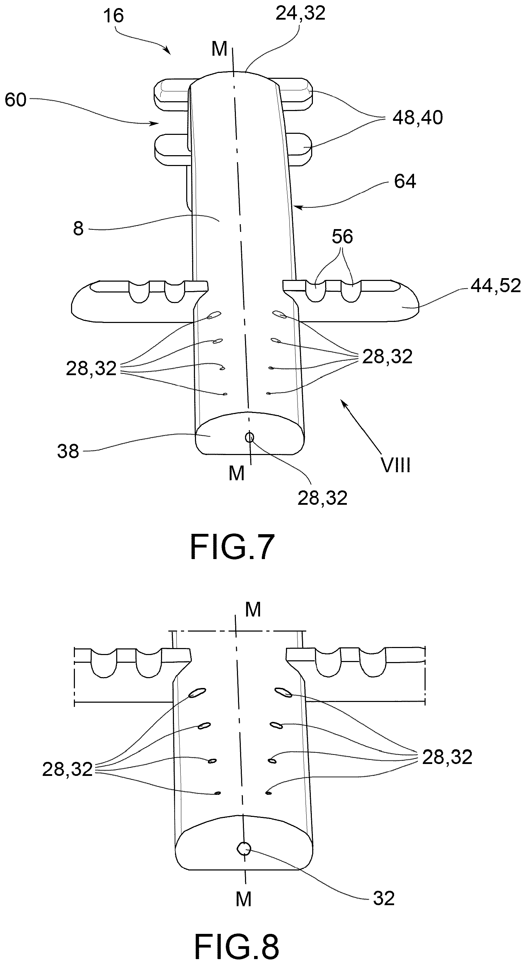

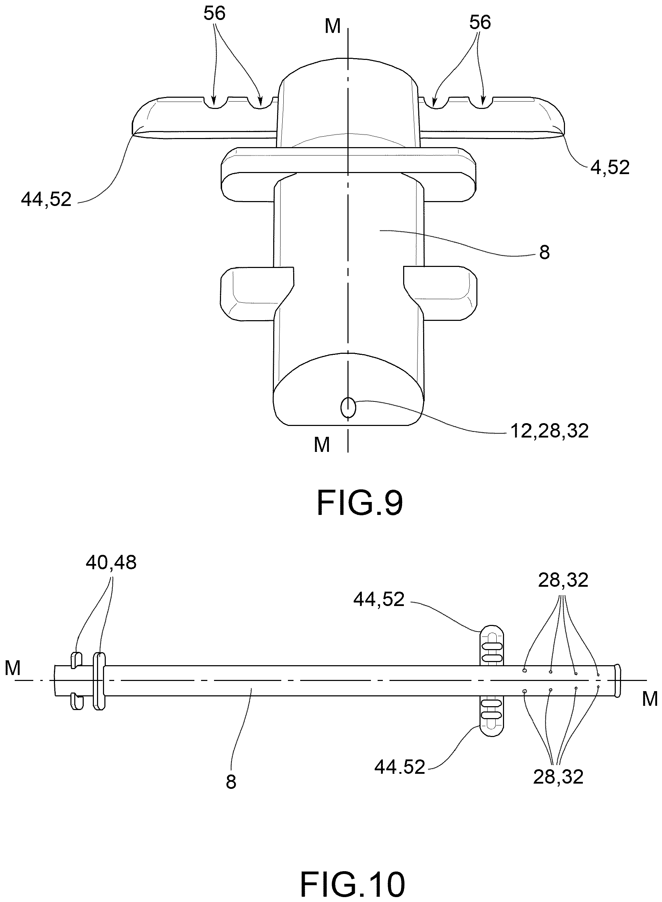

According to a possible embodiment (figures 7-11), the tubular body 8 comprises a plurality of distal filtration holes 28 having different diameters; in particular, said distal filtration holes 28 have an increasing diameter moving from the distal end 20 towards the proximal end 16. Said distal filtration holes 28 with variable diameter allow choosing the quantity of filtration flow based on the pressure of the individual patient. They also allow a progression of the amount of flow, as the ophthalmologist can begin to open the distal filtration hole 28 with a smaller diameter and then subsequently act on, for example, one or two holes of greater diameter if the intraocular pressure conditions require it.

Furthermore, according to a possible embodiment (figures 7-11), said distal filtration holes 28 are located at least partially on opposite sides with respect to a median plane M-M of the tubular body 8 so as to allow, by means of differentiated openings, having a flow directed more to the left or to the right of said median plane M-M of the tubular body 8.

Advantageously, said proximal and distal filtration holes 24, 28 are at least partially or completely occluded by a membrane 32 which can be removed at least partially by selective action, so as to allow the selective variation of the amount of aqueous humour flow removable via said through cavity 12.

According to a possible embodiment, said membrane 32 is a membrane that can be perforated or removed by means of a stylet.

According to a possible embodiment, said membrane 32 is a membrane that can be perforated or removed by application of a laser source from the outside.

According to a further possible embodiment, said membrane 32 is a mobile membrane so as to allow the patency of the corresponding at least one proximal or distal occluding filtration hole 24, 28 to be varied.

According to a further possible embodiment, said membrane 32 is a resorbable membrane in contact with aqueous humor. The reabsorption of the membrane can be established or predetermined in a predetermined time, not being instantaneous. This predetermined time can be varied at will by acting, for example, on the size and/or thickness and/or material of the reabsorbable membrane 32.

It should be noted that the embodiments of membrane 32 described above are not necessarily alternative to each other: in other words, they may coexist in the same embodiment.

For example, the membrane 32 can be perforated and removed by means of a stylet and/or by means of a laser source, and it can also be mobile and/or resorbable in contact with aqueous humor in a time predetermined by the features of the resorbable material and/or by the geometry of the membrane.

All the aforementioned embodiments constitute alternative or synergistic variants which allow exactly dosing or calibrating the overall patency of the medical device 4 and therefore the aqueous humor drainage/filtering capacity.

According to an embodiment, at the proximal end 16, the tubular body 8 comprises a first proximal positioning means 40 of the medical device 4, while, on the side of the distal end 20, the tubular body 8 comprises a second distal positioning means 44 of the medical device 4.

According to an embodiment, the first proximal positioning means 40 of the tubular body 8 comprises a pair of rings or positioning protuberances 48.

According to an embodiment, said rings or positioning protuberances 48 are arranged in positions which are diametrically opposite to the tubular body 8 itself.

For example, said rings or positioning protuberances 48 are axially spaced from each other by a distance of between 0.5 and 3 mm, so as to position themselves astride the sclera-cornea junction. This conformation allows the medical device 4 to remain in place in the irido-sclero-corneal junction, preventing it from being displaced intraocularly or being extruded out of the eye.

According to an embodiment, the second distal positioning means 44 comprises a pair of positioning and attachment tabs 52 for positioning and attaching to the ocular sclera. These positioning and attachment tabs 52 allow the medical device 4 to remain in place, preventing it from moving on the sclera and causing a dislocation thereof with respect to the design working position at the level of the irido-sclero-corneal junction.

Preferably, said positioning and attachment tabs 52 are arranged in positions which are diametrically opposite to the tubular body 8.

According to a possible embodiment, said positioning and attachment tabs 52 are provided with seats 56 for receiving suture threads.

According to a further possible embodiment, said positioning and attachment tabs 52 are smooth, that is, without said seats 56, and are shaped in such a way as to be able to be inserted in scleral pockets, without the need to apply sutures.

Preferably, the rings or positioning protuberances 48 and the positioning and attachment tabs 52 are angularly aligned with each other with respect to the tubular body 8.

According to an embodiment, the tubular body 8 comprises a first proximal tract 60, provided with the first proximal positioning means 40, and a second distal tract 64, provided with the second distal positioning means 44.

According to a possible embodiment, said first proximal portion 60 and second distal portion 64 are rectilinear, respectively, along a first prevailing direction X-X and a second prevailing direction Y-Y, preferably inclined with each other by an angle of deflection 68 between 25 and 45 degrees. In this way, the tubular body 8 is able to position itself radially along the sclera.

According to a further embodiment, the profile of said second distal portion 64 is slightly curved according to the radius of curvature of the sclera in the prevailing direction Y-Y.

For example, the first proximal tract 60 has an extension along the first prevailing direction X-X between 1.5 and 3.5 mm, while the second distal portion 64 has an extension along the second prevailing direction Y-Y between 6 and 9 mm.

The method of use and the operation of the medical device according to the present invention will now be described.

In particular, the device is positioned on the side of its proximal end 16 at the irido-sclero-corneal junction of the patient.

The flow of aqueous humor, exiting the eye through the medical device 4, begins when the reabsorption of the reabsorbable membrane/cap 32 occurs at the level of the endocular hole or the proximal filtration hole 24 (or it is opened by means of a YAG laser) and/or when the reabsorption of the reabsorbable membrane/cap 32 occurs at the level of the distal filtration hole 28 or the opening of at least one of the distal filtration holes 28 is made on the upper or upper-lateral surface 36 of the medical device 4 itself by the methods described above.

The opening of the filtration holes 24, 28 is made directly or occurs by reabsorption of the membrane 32 after a suitable number of days, so as to give the conjunctival wound time to heal and be sealed. The amount or volume of filtration is determined by the diameter and number of openings of the filtration holes 24, 28 on the extraocular segment.

As mentioned above, this opening of the filtration holes 24, 28 may take place in various ways.

For example, the opening of the filtration holes 24, 28 takes place by pressure with a metal stylet (or alternatively also of other materials) thereon so as to obtain the yielding thereof towards the inside of the tubular body 8 according to a programmed, controlled and predetermined process. Each opening of a filtration hole 24, 28 causes the release of a predetermined quantity of microliters of aqueous humor/hour. As seen, it is also possible to provide filtration holes 24, 28 which can be opened by treatment with an argon-type thermal laser or by means of a YAG laser. The material of the holes can be the same as the structure of the medical device 4 or different, according to the needs and opening methods.

The proximal hole 24 of the endocular ending is preferably closed by a membrane of resorbable material and therefore is not patent at the time of implantation. The closure material has the feature of being resorbable in about 1-15 days. Alternatively, the closure may be made with non-resorbable material but sensitive to treatment with a YAG laser which causes the destruction of the material itself and the consequent patency of the medical device 4.

Therefore, the implant does not determine any filtration in the immediate postoperative period (as in known solutions). This specific feature allows the surgery-induced conjunctival wound to heal before the flow of aqueous humor begins.

As can be seen from the above description, the medical device according to the invention allows the drawbacks of the prior art to be overcome.

In particular, the present invention allows modulating the filtration in the postoperative period in a selective, progressive and controlled manner according to the intraocular pressure of the single patient.

Furthermore, the device allows solving the problem of abnormal filtration through the conjunctival suture in the immediate postoperative period.

Furthermore, the device allows solving the problem of abnormal filtration due to the fact that filtration is not immediate, but occurs only after the conjunctiva has healed correctly.

As described above, the suture of the scleral flap is made to be sealed. The aqueous humor does not filter through the scleral flap (as in the solutions of the prior art) but filters through the filtration holes outside the same. The unpredictability caused by the surgeon's hand in placing the sutures of the flap is therefore eliminated. Therefore, in this case the scleral flap has only the protective function of the implanted device and no longer determines the quantity or volume of the aqueous humor flow (as occurs in the solutions of the prior art).

Furthermore, as seen, the positioning tabs allow both the suture of the implant to the sclera and their positioning in scleral pockets without the need for sutures.

A person skilled in the art may make several changes and adjustments to the medical devices described above in order to meet specific and incidental needs, all falling within the scope of protection defined in the following claims.

## Claims

1. A medical device (4) for glaucoma surgery comprising:
- a tubular body (8) with a through cavity (12) extending from a proximal or intraocular end (16) to a distal or extraocular end (20),
- wherein at the proximal end (16) the tubular body (8) comprises a first proximal positioning means (40) of the medical device (4),
- wherein the tubular body (8) comprises at least one proximal filtration hole (24), located on the side of the proximal end (16), and at least one distal filtration hole (28), located on the side of the distal end (20), said proximal and distal filtration holes (24,28), being fluidically connected to each other by means of said through cavity (12),
**characterized in that**
- on the side of the distal end (20), the tubular body (8) comprises a second distal positioning means (44) of the medical device (4),
- wherein said proximal and distal filtration holes (24,28) are at least partially or completely occluded by a membrane (32) that can be removed at least partially by selective action, so as to allow the selective variation of the amount of aqueous humour flow removable via said through cavity (12).

2. The medical device (4) for glaucoma surgery according to claim 1, wherein said membrane (32) is a membrane that can be perforated or removed by means of a stylet and/or by the application of a laser source.

3. The medical device (4) for glaucoma surgery (4) according to any one of claims 1 to 2, wherein said membrane (32) is a mobile membrane so as to allow the patency of the corresponding at least one proximal or distal occluding filtration hole (24, 28) to be varied.

4. The medical device (4) for glaucoma surgery according to any one of claims 1 to 3, wherein said membrane (32) is a membrane resorbable in contact with aqueous humour in a predetermined time range.

5. The medical device (4) for glaucoma surgery according to any of the previous claims, wherein the through cavity (12) has a through width or lumen with an internal diameter between 40 and 140 microns.

6. The medical device (4) for glaucoma surgery according to any one of claims 1 to 5, wherein the at least one proximal filtration hole (24) is located at the proximal end (16) and wherein the at least one distal filtration hole (28) is located on an upper and/or upper-rear and/or upper-lateral surface (36) of the tubular body (8).

7. The medical device (4) for glaucoma surgery according to any one of claims 1 to 6, wherein the tubular body (8) comprises a plurality of distal filtration holes (28) having increasing diameter moving from the distal end (20) towards the proximal end (16).

8. The medical device (4) for glaucoma surgery according to any one of claims 1 to 7, wherein the tubular body (8) comprises a plurality of distal filtration holes (28) located at least partially on opposite sides with respect to a median plane (M-M) of the tubular body 8.

9. The medical device (4) for glaucoma surgery according to any one of claims 1 to 8, wherein the tubular body (8), with respect to a section plane perpendicular to a prevailing direction of extension of the medical device (4) which connects the proximal or intraocular end (16) with the distal or extraocular end (20), has an at least partially concave section (22) at a side or lower face (23) suitable for interfacing with an ocular sclera (25).

10. The medical device (4) for glaucoma surgery according to any one of claims 1 to 9, wherein the tubular body (8), with respect to a section plane perpendicular to a prevailing direction of extension of the medical device (4) which connects the proximal or intraocular end (16) with the distal or extraocular end (20), has a convex, elliptical or parabolic section, at a side or upper face (27) suitable for interfacing with a conjunctiva (29).

11. The medical device (4) for glaucoma surgery according to any one of claims 1 to 10, wherein the first proximal positioning means (40) of the tubular body (8) comprises a pair of rings or positioning protuberances (48) arranged in positions which are diametrically opposite to the tubular body (8) and wherein said rings or positioning protuberances (48) are axially spaced from each other by a distance of between 0.5 mm and 3 mm, so as to position themselves astride the sclera-cornea junction.

12. The medical device (4) for glaucoma surgery according to any one of claims 1 to 11, wherein the second distal positioning means (44) comprise a pair of positioning and attachment tabs (52) for positioning and attaching to the ocular sclera, wherein said positioning and attachment tabs (52) are arranged in positions which are diametrically opposite to the tubular body (8).

13. The medical device (4) for glaucoma surgery according to claim 12, wherein said positioning tabs (52) are provided with seats (56) for receiving suture threads.

14. The medical device (4) for glaucoma surgery according to claim 12, wherein said positioning and attachment tabs (52) are smooth and are shaped so as to be inserted into scleral pockets, without the need to apply sutures.

15. The medical device (4) for glaucoma surgery according to claim 11 in combination with any one of claims 12 to 14, wherein the positioning rings or protuberances (48) and the positioning and attachment tabs (52) are aligned angularly with each other with respect to the tubular body (8).

16. The medical device (4) for glaucoma surgery according to any one of claims 1 to 15, wherein the tubular body (8) comprises a first proximal tract (60), provided with the first proximal positioning means (40), and a second distal tract (64), provided with the second distal positioning means (44), said first and second tract (60, 64) being rectilinear, respectively, along a first and second prevailing direction (X-X, Y-Y), said prevailing directions (X-X, Y-Y) being inclined with each other by an angle of deflection (68) between 25 and 45 degrees.

17. The medical device (4) for glaucoma surgery according to any one of claims 1 to 16, wherein the tubular body (8) comprises a first proximal tract (60), provided with the first proximal positioning means (40), and a second distal tract (64), provided with the second distal positioning means (44), said first and second tracts (60, 64) being directed respectively along a first and a second prevailing direction (X-X, Y-Y), wherein said second distal tract (64) is slightly curved according to the radius of curvature of the sclera in the second prevailing direction (Y-Y).

## Patentansprüche

1. Medizinische Vorrichtung (4) für Glaukomchirurgie, umfassend:
- einen röhrenförmigen Körper (8) mit einem durchgehenden Hohlraum (12), welcher sich von einem proximalen oder intraokularen Ende (16) zu einem distalen oder extraokularen Ende (20) erstreckt,
- wobei an dem proximalen Ende (16) der röhrenförmige Körper (8) ein erstes proximales Positionierungsmittel (40) der medizinischen Vorrichtung (4) umfasst,
- wobei der röhrenförmige Körper (8) wenigstens ein proximales Filtrationsloch (24), welches an der Seite des proximalen Endes (16) positioniert ist, und wenigstens ein distales Filtrationsloch (28) umfasst, welches an der Seite des distalen Endes (20) positioniert ist, wobei die proximalen und die distalen Filtrationslöcher (24, 28) mittels des durchgehenden Hohlraums (12) fluidisch miteinander verbunden sind,
**dadurch gekennzeichnet, dass**
- an der Seite des distalen Endes (20) der röhrenförmige Körper (8) ein zweites distales Positionierungsmittel (44) der medizinischen Vorrichtung (4) umfasst,
- wobei die proximalen und die distalen Filtrationslöcher (24, 28) wenigstens teilweise oder vollständig durch eine Membran (32) verschlossen sind, welche wenigstens teilweise durch eine selektive Aktion entfernt werden kann, um die selektive Variation der Menge einer Kammerwasserströmung zu ermöglichen, welche über den durchgehenden Hohlraum (12) entfernbar ist.

2. Medizinische Vorrichtung (4) für Glaukomchirurgie nach Anspruch 1, wobei die Membran (32) eine Membran ist, welche mittels eines Stiletts und/oder durch die Anwendung einer Laserquelle perforiert oder entfernt werden kann.

3. Medizinische Vorrichtung (4) für Glaukomchirurgie (4) nach einem der Ansprüche 1 bis 2, wobei die Membran (32) eine mobile Membran ist, um zu ermöglichen, dass die Durchgängigkeit des entsprechenden wenigstens einen proximalen oder distalen verschließenden Filtrationslochs (24, 28) variiert wird.

4. Medizinische Vorrichtung (4) für Glaukomchirurgie nach einem der Ansprüche 1 bis 3, wobei die Membran (32) eine Membran ist, welche in Kontakt mit Kammerwasser in einem vorbestimmten Zeitbereich resorbierbar ist.

5. Medizinische Vorrichtung (4) für Glaukomchirurgie nach einem der vorhergehenden Ansprüche, wobei der durchgehende Hohlraum (12) eine durchgehende Breite oder ein durchgehendes Lumen mit einem Innendurchmesser zwischen 40 und 140 Mikrometer umfasst.

6. Medizinische Vorrichtung (4) für Glaukomchirurgie nach einem der Ansprüche 1 bis 5, wobei das wenigstens eine proximale Filtrationsloch (24) an dem proximalen Ende (16) positioniert ist und wobei das wenigstens eine distale Filtrationsloch (28) an einer oberen und/oder oberen hinteren und/oder oberen lateralen Fläche (36) des röhrenförmigen Körpers (8) positioniert ist.

7. Medizinische Vorrichtung (4) für Glaukomchirurgie nach einem der Ansprüche 1 bis 6, wobei der röhrenförmige Körper (8) eine Mehrzahl von distalen Filtrationslöchern (28) umfasst, welche sich von dem distalen Ende (20) in Richtung des proximalen Endes (16) bewegend einen ansteigenden Durchmesser aufweisen.

8. Medizinische Vorrichtung (4) für Glaukomchirurgie nach einem der Ansprüche 1 bis 7, wobei der röhrenförmige Körper (8) eine Mehrzahl von distalen Filtrationslöchern (28) umfasst, welche wenigstens teilweise an entgegengesetzten Seiten in Bezug auf eine Mittelebene (M-M) des röhrenförmigen Körpers (8) positioniert sind.

9. Medizinische Vorrichtung (4) für Glaukomchirurgie nach einem der Ansprüche 1 bis 8, wobei der röhrenförmige Körper (8) in Bezug auf eine Schnittebene senkrecht zu einer vorherrschenden Erstreckungsrichtung der medizinischen Vorrichtung (4), welche das proximale oder intraokulare Ende (16) mit dem distalen oder extraokularen Ende (20) verbindet, einen wenigstens teilweise konkaven Abschnitt (22) an einer Seite oder einer unteren Fläche (23) aufweist, welche dazu geeignet ist, eine Schnittstelle mit einer okularen Sklera (25) zu bilden.

10. Medizinische Vorrichtung (4) für Glaukomchirurgie nach einem der Ansprüche 1 bis 9, wobei der röhrenförmige Körper (8) in Bezug auf eine Schnittebene senkrecht zu einer vorherrschenden Erstreckungsrichtung der medizinischen Vorrichtung (4), welche das proximale oder intraokulare Ende (16) mit dem distalen oder extraokularen Ende (20) verbindet, einen konvexen, elliptischen oder parabolischen Abschnitt an einer Seite oder einer oberen Fläche (27) aufweist, welche dazu geeignet ist, eine Schnittstelle mit einer Bindehaut (29) zu bilden.

11. Medizinische Vorrichtung (4) für Glaukomchirurgie nach einem der Ansprüche 1 bis 10, wobei das erste proximale Positionierungsmittel (40) des röhrenförmigen Körpers (8) ein Paar von Ringen oder Positionierungsvorsprüngen (48) umfasst, welche in Positionen angeordnet sind, die zu dem röhrenförmigen Körper (8) diametral entgegengesetzt sind, und wobei die Ringe oder Positionierungsvorsprünge (48) in einem Abstand von zwischen 0,5 mm und 3 mm axial voneinander beabstandet sind, um sich selbst rittlings der Sklera-Cornea-Verbindung zu positionieren.

12. Medizinische Vorrichtung (4) für Glaukomchirurgie nach einem der Ansprüche 1 bis 11, wobei das zweite distale Positionierungsmittel (44) ein Paar von Positionierungs- und Anbringungszungen (52) zum Positionieren und Anbringen der okularen Sklera umfasst, wobei die Positionierungs- und Anbringungszungen (52) in Positionen angeordnet sind, welche zu dem röhrenförmigen Körper (8) diametral entgegengesetzt sind.

13. Medizinische Vorrichtung (4) für Glaukomchirurgie nach Anspruch 12, wobei die Positionierungszungen (52) mit Aufnahmen (56) zum Aufnehmen von Nähfäden bereitgestellt sind.

14. Medizinische Vorrichtung (4) für Glaukomchirurgie nach Anspruch 12, wobei die Positionierungs- und Anbringungszungen (52) glatt sind und geformt sind, um ohne die Notwendigkeit, Nähte zu verwenden, in sklerale Taschen eingesetzt zu sein.

15. Medizinische Vorrichtung (4) für Glaukomchirurgie nach Anspruch 11 in Kombination mit jeglichem der Ansprüche 12 bis 14, wobei die Positionierungsringe oder Vorsprünge (48) und die Positionierungs- und Anbringungszungen (52) in Bezug auf den röhrenförmigen Körper (8) winkelmäßig zueinander ausgerichtet sind.

16. Medizinische Vorrichtung (4) für Glaukomchirurgie nach einem der Ansprüche 1 bis 15, wobei der röhrenförmige Körper (8) einen ersten proximalen Trakt (60), welcher mit dem ersten proximalen Positionierungsmittel (40) bereitgestellt ist, und einen zweiten distalen Trakt (64) umfasst, welcher mit dem zweiten distalen Positionierungsmittel (44) bereitgestellt ist, wobei der erste und der zweite Trakt (60, 64) entlang einer ersten bzw. einer zweiten vorherrschenden Richtung (X-X, Y-Y) geradlinig sind, wobei die vorherrschenden Richtungen (X-X, Y-Y) um einen Ablenkwinkel (68) zwischen 25 und 45 Grad zueinander geneigt sind.

17. Medizinische Vorrichtung (4) für Glaukomchirurgie nach einem der Ansprüche 1 bis 16, wobei der röhrenförmige Körper (8) einen ersten proximalen Trakt (60), welcher mit dem ersten proximalen Positionierungsmittel (40) bereitgestellt ist, und einen zweiten distalen Trakt (64) umfasst, welcher mit dem zweiten distalen Positionierungsmittel (44) bereitgestellt ist, wobei der erste und der zweite Trakt (60, 64) entlang einer ersten bzw. einer zweiten vorherrschenden Richtung (X-X, Y-Y) gerichtet sind, wobei der zweite distale Trakt (64) gemäß dem Krümmungsradius der Sklera in der zweiten vorherrschenden Richtung (Y-Y) leicht gekrümmt ist.

## Revendications

1. Dispositif médical (4) pour la chirurgie du glaucome comprenant :
- un corps tubulaire (8) présentant une cavité traversante (12) s'étendant depuis une extrémité proximale ou intraoculaire (16) jusqu'à une extrémité distale ou extra-oculaire (20),
- dans lequel, au niveau de l'extrémité proximale (16), le corps tubulaire (8) comprend un premier moyen de positionnement proximal (40) du dispositif médical (4),
- dans lequel le corps tubulaire (8) comprend au moins un orifice de filtration (24) proximal, situé sur le côté de l'extrémité proximale (16), et au moins un orifice de filtration (28) distal, situé sur le côté de l'extrémité distale (20), lesdits orifices de filtration (24, 28) proximal et distal étant reliés de manière fluidique l'un à l'autre au moyen de ladite cavité traversante (12),
**caractérisé en ce que**
- sur le côté de l'extrémité distale (20), le corps tubulaire (8) comprend un deuxième moyen de positionnement distal (44) du dispositif médical (4),
- dans lequel lesdits orifices de filtration proximal et distal (24, 28) sont au moins partiellement ou complètement occlus par une membrane (32) qui peut être retirée, au moins partiellement, par une action sélective, de manière à permettre la variation sélective de la quantité d'écoulement d'humeur aqueuse pouvant être retirée via ladite cavité traversante (12).

2. Dispositif médical (4) pour la chirurgie du glaucome selon la revendication 1, dans lequel ladite membrane (32) est une membrane qui peut être perforée ou retirée au moyen d'un stylet et/ou par l'application d'une source laser.

3. Dispositif médical (4) pour la chirurgie du glaucome (4) selon l'une quelconque des revendications 1 à 2, dans lequel ladite membrane (32) est une membrane mobile de manière à pouvoir faire varier la perméabilité de l'orifice de filtration proximal ou distal (24, 28) avec occlusion correspondant, au moins au nombre de un.

4. Dispositif médical (4) pour la chirurgie du glaucome selon l'une quelconque des revendications 1 à 3, dans lequel ladite membrane (32) est une membrane résorbable en contact avec une humeur aqueuse dans un intervalle de temps prédéterminé.

5. Dispositif médical (4) pour la chirurgie du glaucome selon l'une quelconque des revendications précédentes, dans lequel la cavité traversante (12) a une largeur traversante ou une lumière présentant un diamètre interne compris entre 40 et 140 microns.

6. Dispositif médical (4) pour la chirurgie du glaucome selon l'une quelconque des revendications 1 à 5, dans lequel l'orifice de filtration proximal (24), au moins au nombre de un, est situé à l'extrémité proximale (16), et dans lequel l'orifice de filtration distal (28), au moins au nombre de un, est situé sur une surface supérieure et/ou arrière supérieure et/ou latérale supérieure (36) du corps tubulaire (8).

7. Dispositif médical (4) pour la chirurgie du glaucome selon l'une quelconque des revendications 1 à 6, dans lequel le corps tubulaire (8) comprend une pluralité d'orifices de filtration (28) distaux présentant un diamètre croissant en allant de l'extrémité distale (20) vers l'extrémité proximale (16).

8. Dispositif médical (4) pour la chirurgie du glaucome selon l'une quelconque des revendications 1 à 7, dans lequel le corps tubulaire (8) comprend une pluralité d'orifices de filtration distaux (28) situés au moins partiellement sur des côtés opposés par rapport à un plan médian (M-M) du corps tubulaire (8).

9. Dispositif médical (4) pour la chirurgie du glaucome selon l'une quelconque des revendications 1 à 8, dans lequel le corps tubulaire (8), par rapport à un plan de coupe perpendiculaire à une direction dominante d'extension du dispositif médical (4) qui relie l'extrémité proximale ou intraoculaire (16) à l'extrémité distale ou extra-oculaire (20), présente une section au moins partiellement concave (22) au niveau d'un côté ou d'une face inférieure (23), appropriée pour assurer une interface avec une sclère oculaire (25).

10. Dispositif médical (4) pour la chirurgie du glaucome selon l'une quelconque des revendications 1 à 9, dans lequel le corps tubulaire (8), par rapport à un plan de coupe perpendiculaire à une direction dominante d'extension du dispositif médical (4) qui relie l'extrémité proximale ou intraoculaire (16) à l'extrémité distale ou extra-oculaire (20), présente une section convexe, elliptique ou parabolique, au niveau d'un côté ou d'une face supérieure (27), appropriée pour assurer une interface avec une conjonctive (29).

11. Dispositif médical (4) pour la chirurgie du glaucome selon l'une quelconque des revendications 1 à 10, dans lequel le premier moyen de positionnement proximal (40) du corps tubulaire (8) comprend une paire d'anneaux ou protubérances de positionnement (48) agencés dans des positions qui sont diamétralement opposées au corps tubulaire (8), et dans lequel lesdits anneaux ou protubérances de positionnement (48) sont espacés axialement les uns des autres d'une distance comprise entre 0,5 mm et 3 mm, de manière à se positionner eux-mêmes à cheval sur la jonction de la sclère et de la cornée.

12. Dispositif médical (4) pour la chirurgie du glaucome selon l'une quelconque des revendications 1 à 11, dans lequel les deuxièmes moyens de positionnement distaux (44) comprennent une paire de pattes de positionnement et de fixation (52) permettant le positionnement et la fixation à la sclère oculaire, dans lequel lesdites pattes de positionnement et de fixation (52) sont agencées dans des positions qui sont diamétralement opposées au corps tubulaire (8).

13. Dispositif médical (4) pour la chirurgie du glaucome selon la revendication 12, dans lequel lesdites pattes de positionnement (52) sont dotées de sièges (56) permettant de recevoir des fils de suture.

14. Dispositif médical (4) pour la chirurgie du glaucome selon la revendication 12, dans lequel lesdites pattes de positionnement et de fixation (52) sont lisses et sont formées de manière à être insérées dans des poches sclérales sans qu'il soit nécessaire d'appliquer des sutures.

15. Dispositif médical (4) pour la chirurgie du glaucome selon la revendication 11 en association avec l'une quelconque des revendications 12 à 14, dans lequel les anneaux ou protubérances de positionnement (48) et les pattes de positionnement et de fixation (52) sont alignés angulairement les uns sur les autres par rapport au corps tubulaire (8).

16. Dispositif médical (4) pour la chirurgie du glaucome selon l'une quelconque des revendications 1 à 15, dans lequel le corps tubulaire (8) comprend une première voie proximale (60), dotée du premier moyen de positionnement proximal (40), et une deuxième voie distale (64), dotée du deuxième moyen de positionnement distal (44), lesdites première et deuxième voies (60, 64) étant rectilignes, respectivement, le long d'une première et d'une deuxième directions dominantes (X-X, Y-Y), lesdites directions dominantes (X-X, Y-Y) étant inclinées l'une par rapport à l'autre selon un angle de déviation (68) compris entre 25 et 45 degrés.

17. Dispositif médical (4) pour la chirurgie du glaucome selon l'une quelconque des revendications 1 à 16, dans lequel le corps tubulaire (8) comprend une première voie proximale (60), dotée du premier moyen de positionnement proximal (40), et une deuxième voie distale (64), dotée du deuxième moyen de positionnement distal (44), lesdites première et deuxième voies (60, 64) étant dirigées respectivement le long d'une première et d'une deuxième directions dominantes (X-X, Y-Y), ladite deuxième voie distale (64) étant légèrement incurvée conformément au rayon de courbure de la sclère dans la deuxième direction dominante (Y-Y).
